# EUROPEAN PATENT APPLICATION

(11) **EP 2 418 285 A1**
(43) Date of publication of application: **15.02.2012**
(21) Application number: 10172295.7
(22) Date of filing: 09.08.2010
(51) Int. Cl.: C12Q 1/42, G01N 33/68

(54) **Method for diagnosing fatty liver diseases, in particular non-alcoholic steatohepatitis.**

(71) Applicant: Universiteit Maastricht, 6211 LK Maastricht (NL); Academisch Ziekenhuis Maastricht, 6229 HX Maastricht (NL)
(72) Inventor: Sverdlov, Ronit, 6228 SB, Maastricht (NL); Bieghs, Veerle, 3650, Dilsen (BE); Gorp, Van, Patrick Johannes Jacobus, 6301 LR, Valkenburg a/d Geul (NL)
(74) Representative: Habets, Winand

(57) **Abstract**

The invention is in the field of medical diagnostics of liver disease, in particular non-alcoholic fatty liver diseases, such as non-alcoholic steatohepatitis. The invention provides a method and compositions for the diagnosis of NASH in that the levels of acid phosphatase activity in a sample obtained from a subject are determined.

## Description

### Field of the invention

The invention is in the field of diagnosis of liver diseases, in particular fatty liver disease, more in particular non-alcoholic fatty liver disease, such as non-alcoholic steatohepatitis.

### Background of the invention

Non alcoholic fatty liver disease (NAFLD) is a condition ranging from benign lipid accumulation in the liver (steatosis) to steatosis combined with inflammation. The latter is referred to as non-alcoholic steatohepatitis (NASH). NASH is viewed as the hepatic event of the metabolic syndrome. Estimates in USA are that 5.7-17% of all adults have NASH, while 17% to 33% of Americans have NAFLD. NASH and NAFLD are frequently reported in both men and women, although it most often appears in women and is especially prevalent in the obese population. As obesity and insulin resistance reach epidemic proportions in industrialized countries, the prevalence of both NAFLD and NASH is increasing and is therefore considered as a major health hazard.

There is no established therapy for patients suffering from NASH. Therapy is, therefore, focused mainly on risk factors, weight reduction and pharmacological intervention. Promising pharmacological treatments have been demonstrated with antioxidants, insulin sensitizers, hepatoprotectants and lipidlowering agents.

Steatosis alone is considered a relatively benign and reversible condition. However, the transition towards NASH represents a key step in the pathogenesis, as it will set the stage for further damage to the liver, such as fibrosis, cirrhosis and liver cancer. At present, available non-invasive markers for steatosis include a set of clinical symptoms and combinations of blood tests. Although several of these markers are useful in the diagnostic evaluation of a patient with steatosis, they lack specificity and sensitivity to distinguish NASH from simple steatosis. This situation represents a key clinical problem, as patients with NASH are those that need close monitoring and frequent follow-up. Currently, liver biopsy is used as the primary method for detection of liver inflammation in clinical practice.

Despite the fact that a number of biomarkers for NASH have been suggested recently (reviewed in Wieckowska et al., Hepatology 46, 582-589 (2007)), liver biopsy is used in the clinical practice as the primary method for detection of liver inflammation in NASH. There is a great need in the art for simple, reproducible and reliable non-invasive test that can distinguish steatosis from NASH.

### Summary of the invention

The present invention provides methods and compositions for the diagnosis of non-alcoholic steatohepatitis, (NASH). They may not only help in the diagnosis of NASH but also be useful for assessment of treatment response and prognosis.

We have used Ldlr knock-out mice that were transplanted with bone marrow from mice lacking *Cyp27A1.* Control mice were transplanted with wild-type (Wt) bone marrow. After a recovery period of 9 weeks, the mice were put on chow or high fat high cholesterol (HFC) diet for 3 months.

Liver histology demonstrated that *Cyp27A1*^{*-*/*-*}-tp mice had increased hepatic inflammation compared to VVt-tp mice and reduced acid phosphatase activity in the plasma. Both groups of mice which were fed with a normal (Chow) diet did not show any differences, neither in hepatic inflammation nor in acid phosphatase activity levels. It is therefore concluded that acid phosphatase is a marker for liver inflammation.

The invention therefore relates to a method for distinguishing between steatosis and non-alcoholic steatohepatitis wherein the acid phosphatase activity in a sample obtained from a subject is determined and compared to a predetermined reference value and wherein it is concluded that the subject has a higher probability to acquire or suffer from non-alcoholic steatohepatitis when the activity of acid phospatase is below the reference value.

### Legend to the figures

Figure 1: Graphs showing that hepatic inflammation and liver damage is increased in mice transplanted with bone marrow from Cyp27A1 knock out mice, as indicated by the increased number of inflammatory cells and ALT levels.
Figure 2: Graphs showing that hepatic inflammation and liver damage is increased in mice transplanted with bone marrow from Cyp27A1 knock out mice as indicated by the increased gene expression of cytokines.

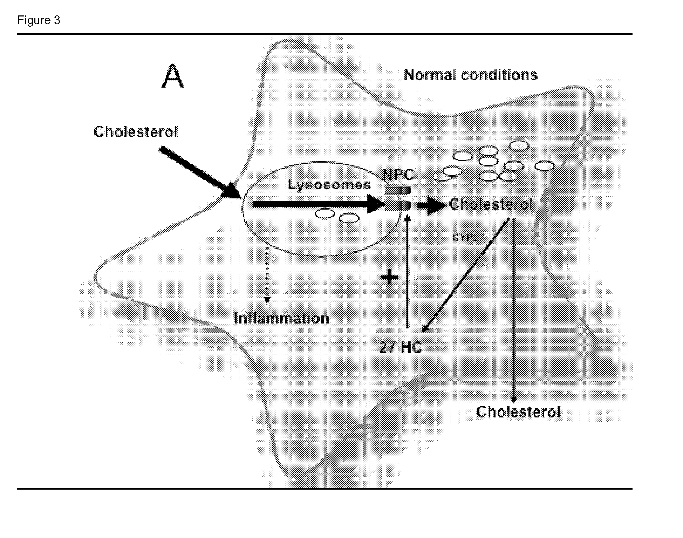

Figure 3: Model showing the role of 27-HC in reducing the levels of lysosomal cholesterol levels.
Figure 4: Graph showing that acid phosphatase activity levels are decreased in mice transplanted with bone marrow from Cyp27A1 knock out mice and fed with a high fat high cholesterol diet as compared to acid phosphatase activity levels of mice transplanted with wild-type bone marrow. Note the absence of such difference in mice fed with a normal Chow diet.

### Detailed description of the invention

Currently, the mechanisms that drive hepatic inflammation during the progression of NASH are largely unknown. We found that lysosomal fat accumulation in Kupffer cells is the underlying mechanism of inflammation in non-alcoholic fatty liver disease, in particular in non-alcoholic steatohepatitis (NASH).

Our study shows for the first time that liver inflammation is induced when 27-HC levels are decreased. We were also able to show that increased levels of 27-HC remedied the problem of lysosomal cholesterol accumulation and that liver inflammation could be ameliorated or cured by increasing 27-HC levels in vivo.

Our model may therefore represent a viable model for studying liver inflammation in NASH and provide new insights in the mechanisms underlying stearohepatitis.

We observed that decreased acid phosphatase activity levels in plasma from mice fed with a high fat high cholesterol diet correlated with hepatic inflammation. It is therefore concluded that acid phosphatase activity is a marker for liver inflammation.

The invention therefore relates to a method for distinguishing between steatosis and non-alcoholic steatohepatitis wherein the acid phosphatase activity in a sample obtained from a subject is determined and compared to a predetermined reference value and wherein it is concluded that the subject has a higher probability to acquire or suffer from non-alcoholic steatohepatitis when the activity of acid phospatase is below the reference value.

The activity of acid phosphatase is an art-recognized term and it may be determined by any suitable method known in the art. For example by the commercially available assay from Sigma-Aldrich, 3050 Spruce Street, St. Louis, MO 63103 USA.

Acid Phosphatase is one of the acid hydrolases that normally reside in lysosomes. It is a classical marker for the identification of lysosomes in subcellular fractionations. The Aldrich Acid Phosphatase Assay Kit is designed for the detection of acid phosphatase activity in whole cell and tissue extracts, column fractions, and purified enzyme preparations. The kit contains all the reagents required for fast and simple acid phosphatase detection. The kit contains a standard solution and a control enzyme. Other methods may be equally suited.

The term "predetermined reference value" is usually also referred to as cut-off value or threshold value and means a value empirically determined or calculated to distinguish the positive from the negative values, or as the case may be, the high from the low values. A reference value may be a value obtained from observations or measurements in a normal population or in a population lacking the feature the test is meant to measure. More in particular, the reference value for determining whether an acid phosphatase level is indicative of steatohepatitis may be an average value obtained from performing a method according to the invention on a sufficient number of individuals with comparable BMI values and/or comparable cholesterol levels as the test subject.

In this particular case, the subject is at a higher risk of acquiring or suffering from steatohepatitis when the acid phosphatase activity in a sample from a test individual is below the average acid phosphatase activity value obtained from individuals not suffering from steatohepatitis.

The reference value may vary depending on the particular methods used to determine the acid phosphatase activity values, however, it is within the reach of a skilled person to determine that activity and reference value with the guidance provided herein, in particular the experiments described in the Examples section

The method according to the invention may be used for the diagnosis of the diseases mentioned herein but may also be used prophylactic, i.e. in order to prevent the diseases from occurring or ameliorating the inflammatory effects once the disease occurs by providing an early diagnosis upon which early treatment may prevent the disease from occurring.

The term "NAFLD" (non-alcohol fatty liver disease) refers to a group of conditions where there is an accumulation of excess fat in the liver of people who drink little or no alcohol. In fatty liver disease, fat accumulates in the liver cells. A subgroup of people with NAFLD may have a more serious condition termed non-alcoholic steatohepatitis (NASH). In NASH, fat accumulation is associated with liver cell inflammation and different degrees of scarring. Cirrhosis occurs when the liver sustains substantial damage, and the liver cells are gradually replaced by scar tissue which results in the inability of the liver to work properly. The use of the term "NAFLD" is used to include all conditions reflecting a form of non-alcohol fatty liver disease, but in particular, NASH.

NAFLD and NASH in particular, involve the development of histologic changes in the liver that are comparable to those induced by excessive alcohol intake but in the absence of alcohol abuse. Macrovesicular and/or microvesicular steatosis, lobular and portal inflammation, and occasionally Mallory bodies with fibrosis and cirrhosis characterize NAFLD. NAFLD (and NASH in particular) is also commonly associated with hyperlipidemia, obesity, hypertension, atherosclerosis, and Type II diabetes mellitus, commonly known as The Metabolic Syndrome. Other clinical conditions characterized by hepatic steatosis and inflammation include excessive fasting, jejunoileal bypass, total parental nutrition, chronic hepatitis C, Wilson's disease, and adverse drug effects such as those from corticosteroids, calcium channel blockers, high dose synthetic estrogens, methotrexate and amiodarone. Thus, the term "non-alcoholic steatohepatitis" can be used to describe those patients who exhibit these biopsy findings, coupled with the absence of (a) significant alcohol consumption, (b) previous surgery for weight loss, (c) history of drug use associated with steatohepatitis, (d) evidence of genetic liver disease or (e) chronic hepatitis C infection. See, J. R. Ludwig et al., Mayo Clin. Proc, 55, 434 (1980) and E. E. Powell et al., Hepatol., 11, 74 (1990).

The phrases "method for the diagnosis" or "method for distinguishing between steatosis and non-alcoholic steatohepatitis" as used herein is meant to refer to in vitro or ex vivo methods that may be performed in the absence of the test subject, in particular an animal or human being. In particular, the methods may be performed on a sample isolated from the human or animal body. The step of isolating the sample is not an essential part of the method as described herein.

Hepatic steatosis (fatty liver) and steatohepatitis (fatty liver with inflammation or scarring) are two forms of the fatty liver disease that develops in children and adults, frequently associated with being overweight or obese. This type of liver disease is often referred to as non-alcoholic steatohepatitis (NASH), because it occurs in the absence of a significant amount of alcohol intake.

### Example 1: Link between uptake of modified lipids and hepatic inflammation.

To evaluate the involvement of uptake of modified lipoproteins by Kupffer cells (Cs) in the development of diet-induced NASH, female low-density lipoprotein receptor-deficient knock-out mice were lethally irradiated and transplanted with bone marrow from Msr1/Cd36 double knock-out mice or WT mice and fed a Western diet. Macrophage and neutrophil infiltration revealed that hepatic inflammation was substantially reduced by approximately 30% in Msr1/Cd36 double knock out transplanted mice compared with control mice. Consistent with this, the expression levels of well-known inflammatory mediators were reduced.

Apoptotis and fibrosis were less pronounced in Msr1/Cd36 double knock out transplanted mice, in addition to the protective phenotype of natural antibodies against oxidized low-density lipoprotein in the plasma. Surprisingly, the effect on hepatic inflammation was independent of foam cell formation.

It was concluded that it is not the total amount of lipids in Kupffer cells that will trigger inflammation, but rather the type of lipids or the mechanism of internalization.

### Example 2: Link between hepatic inflammation in NASH and the intracellular fat localization in Kupffer cells.

Ldlr knock out mice were depleted from their hematopoietic system by irradiation and were transplanted with bone marrow from mice lacking either Sr-a or Cd36. Control mice were injected with wild-type (Wt) bone marrow. After a recovery period of 9 weeks, the mice were put on a high fat diet (HFD) for 3 months.

It was found that Cd36 knock out and Sr-a⁻ knock out transplanted (tp) mice showed reduced inflammatory markers in blood and liver compared to Wt-tp mice. Moreover, fibrosis was also less pronounced in both Cd36 knock out and Sr-a⁻ knock out tp mice compared to Wt-tp mice. In plasma, the protective IgM auto-antibodies were increased in both Cd36 knock out and Sr-a knock out -tp mice compared to Wt-tp mice. Despite the lack of difference in the appearance of foamy Kupffer cells (KC's), electron microscopy (EM) revealed a significant difference in lysosomal/cytoplasmic cholesterol storage. It was observed that the Wt-tp mice showed increased lysosomal fat whereas the Cd36 knock out - and Sr-a⁻ knock out tp mice showed more cytoplasmic fat inside the KC's.

It was concluded that the reduction in inflammation is correlated with decreased lysosomal cholesterol accumulation in Kupffer cells. These observations establish the link between lysosomal fat accumulation in KC's and hepatic inflammation.

### Example 3: expression of CYP27A1 in Kupffer cells can modulate lysosomal fat accumulation in vitro.

We incubated bone marrow derived macrophages from Cyp27A1 knock-out mice and Wt mice with ox LDL for 24 hours. It was found that cholesterol levels in lysosomes were increased. Consequently, cholesterol was significantly more crystallized in macrophages of the Cyp27A1 knock out mice compared to Wt mice.

Since the breakdown of free cholesterol by Cyp27A1 results in the formation of 27-hydroxycholesterol it may be concluded that 27-HC can reduce lysosomal cholesterol accumulation in macrophages.

### Example 4: expression of CYP27A1 in Kupffer cells can modulate lysosomal fat accumulation and thereby hepatic inflammation.

Ldlr knock-out mice were transplanted with bone marrow from mice lacking *Cyp27A1.* Control mice were transplanted with wild-type (Wt) bone marrow. After a recovery period of 9 weeks, the mice were put on chow or high fat high cholesterol (HFC) diet for 3 months.

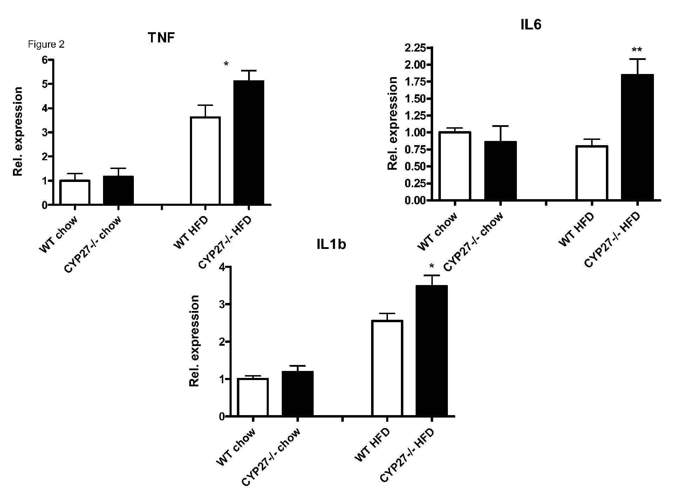

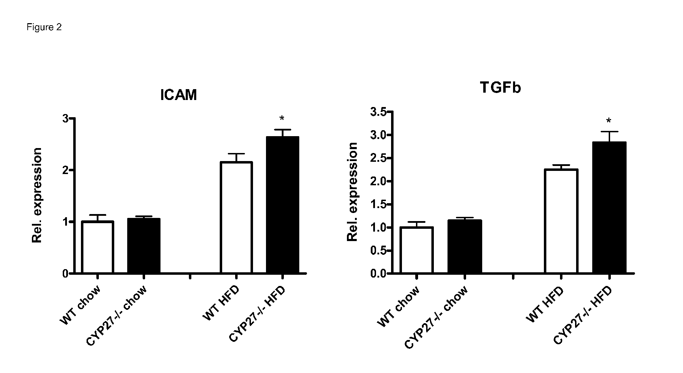

It was found that Kupffer cells of the *Cyp27A1*^{-/-}tp mice showed increased lysosomal fat accumulation. Additionally, cholesterol was more crystallized in *Cyp27A1*^{*-*/*-*}-tp mice compared to *Wt*-tp mice. Surprisingly, CD68 staining revealed that KC's were less foamy upon HFC feeding in *Cyp27A1*^{*-*/*-*}-tp mice. Liver histology demonstrated that *Cyp27A1*^{*-*/*-*}-tp mice had increased hepatic inflammation compared to *Wt*-tp mice as indicated by the elevated numbers of infiltrated macrophages, neutrophils and T-cells. These findings were confirmed by hepatic expression of *Tnf, II-1b* and *II-6 (**Figure 2**).*

It was concluded that CYP27A1 plays an important role in liver inflammation in NASH, in particular in diet-induced NASH.

It may therefore be concluded that there is a clear association between lysosomal fat accumulation and hepatic inflammation and that inflammation may be decreased by increasing the level or activity of 27-HC.

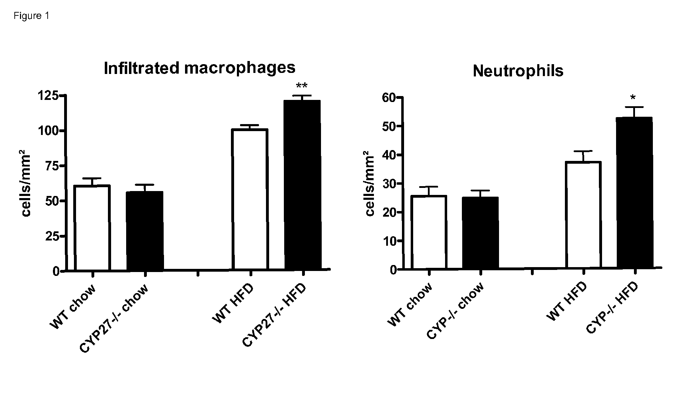

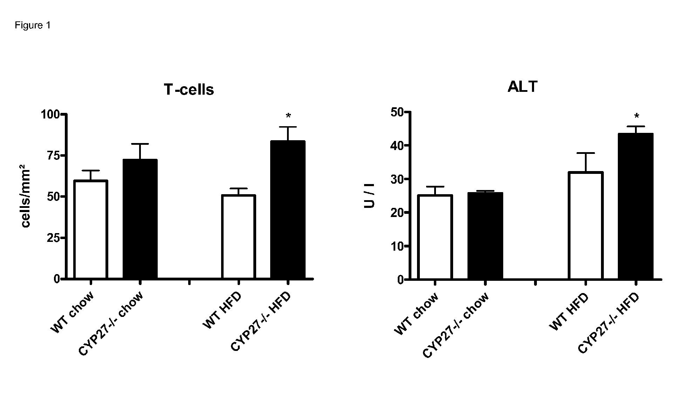

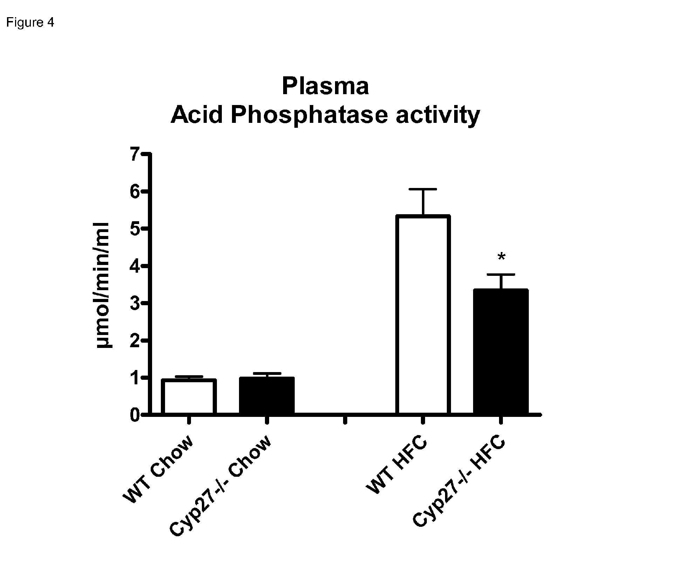

It was also observed that mice fed with a high fat high cholesterol diet showed a marked difference in acid phosphatase levels whereas mice fed with a normal diet did not show any difference (Figure 4). The decreased activity of acid phosphatase, in mice fed a high cholesterol diet, correlated nicely with increased inflammation as indicated by the number of inflammatory cells (figure1) as well as cytokine levels (Figure 2).

### Example 5: Materials and methods used in the other examples

### Mice

Female *Ldlr*^{*-*/*-*} mice were lethally irradiated and transplanted with *Wt, Cd36*^{*-*/*-*} and *Msr*^{*-*/-}bone marrow. After a recovery period of 9 weeks, the mice were given a HFC diet for 3 months (n=9 in Wt-tp mice, n=8 in both Cd36-/--tp and Msr1-/--tp mice).

Collection of blood, specimens, biochemical determination of lipids in plasma and liver, liver histology, RNA isolation, cDNA synthesis and qPCR, determination of chimerism, aminotransferases, oxysterols and auto-antibody titers against modified LDL are described previously (Bieghs, V., et al.,. Gastroenterology, 2010).

### Blood leukocyte analysis

Sixty microliter of total blood was washed extensively in erythrocyte lysis buffer (0.155 M NH₄CL, 10 mM NaHCO₃) for leukocyte analysis. Cells were stained for monocytes (Mac1-PE), granulocytes (Gr1-FITC), B-cells (6B2-PE) and T-cells (KT3-FITC) (BD Sciences Pharmingen, San Diego, California, USA) in PBS containing 5% normal mouse serum and 1% FCS. After 1 hour of incubation, cells were washed and analyzed by FACS analysis (Facssort, BD Sciences Pharmingen).

### Electron microscopy

Detailed overview about (post)fixation, embedding, cutting and type of electron microscope is described previously (Hepatology). Scoring of the electron microscopy pictures is performed by a trained specialist. Fifty Kupffer cells per mouse were analyzed and scored. The Kupffer cells were divided into five different categories, e.g. primary lysosomes, secondary lysosomes, fatty lysosomes, cytoplasmic fat accumulation and degenerated lysosomes and the percentage of each subtype is calculated. Furthermore, the secondary lysosomes were scored from 0 to 3 for their fat content and complexicity, where score 0 indicates not positive and score 3 indicates extreme fat accumulation inside the lysosomes of the KCs / extremely complex indicated by electrodense particles. The same scoring index is used for the cytoplasmic fat accumulation inside Kupffer cells.

### Statistical analysis

Data was statistically analyzed by performing one-way ANOVA test with a Bonferroni post-test using GraphPad Prism for comparing *Wt*-tp, *Cd36*^{*-*/*-*}-tp and *Msr*^{*-*/*-*}-tp mice with each other. Data were expressed as the mean ± SEM and considered significant at p<0.05. *, ** and *** indicate p < 0.05, 0.01 and 0.001 resp.

### Acid Phosphatase assay

Acid Phosphatase in mouse plasma was determined using the Acid Phosphatase Assay Kit from Cayman Chemical Company with Catalog No. 10008051 according to the manufacturer's instructions.

## Claims

1. Method for distinguishing between steatosis and non-alcoholic steatohepatitis
wherein the acid phosphatase activity in a sample obtained from a subject is determined and compared to a predetermined reference value and wherein it is concluded that the subject has a higher probability to acquire or suffer from non-alcoholic steatohepatitis when the activity of acid phospatase is below the reference value.

2. Method according to claim 1 wherein the sample is a plasma sample.

3. Method according to claims 1 or 2 wherein the predetermined reference value is the acid phosphatase level of at least one control individual with a comparable BMI index or a comparable cholesterol level as the subject.
